# EUROPEAN PATENT APPLICATION

(11) **EP 2 204 202 A1**
(43) Date of publication of application: **07.07.2010**
(21) Application number: 08838161.1
(22) Date of filing: 10.10.2008
(51) Int. Cl.: A61M 5/142

(54) **MEDICINAL SOLUTION DISPENSER**

(30) Priority: 10.10.2007 JP 2007264775; 10.10.2007 JP 2007264776; 10.10.2007 JP 2007264777; 24.03.2008 JP 2008076727
(71) Applicant: Olympus Corporation, Tokyo 151-0072 (JP)
(72) Inventor: TOGAWA, Tsuyoshi, Tokyo 151-0072 (JP)
(74) Representative: Gunzelmann, Rainer
(86) International application number: PCT/JP2008/068490
(87) International publication number: WO 2009/048144

(57) **Abstract**

Provided are a medical-solution reservoir that is filled with at least a medical solution and that feeds the medical solution to a medical solution administration side; a pump that discharges to the medical-solution reservoir a driving liquid that is used in pushing the medical solution filled in the medical-solution reservoir; and a driving liquid reservoir that is filled with the driving liquid. The medical-solution reservoir forms a pipe in which the driving liquid discharged by the pump enters from an opening on one end so that the medical solution is pushed to the medical solution administration side from an opening on other end of the pipe. A marker is placed between the driving liquid and the medical solution, the marker being used in detecting a pushed quantity of the medical solution pushed by the driving liquid. As a result, it becomes possible to know the state of medical solution administration and administer a definite quantity of the medical solution that needs to be administered within a predetermined time period.

## Description

### TECHNICAL FIELD

The present invention relates to a medical-solution administration device that enables administration of a medical solution, such as an anticancer drug, to an affected area directly and continuously over a relatively long time period.

### BACKGROUND ART

Conventionally, an osmotic pump (see Non-patent Document 1) has been used to administer a medical solution of a small quantity of about 10 ml in a continuous manner over a relatively long and predetermined period of, for example, about one week.

Non-patent Document 1: Theewes F and Yum SI. Principles of the operation of genericosmotic pump for the delivery of semisolid or liquid drug formulations. Ann Biomed Eng 1976, 4(4):343-353

### DISCLOSURE OF INVENTION

### PROBLEM TO BE SOLVED BY THE INVENTION

Meanwhile, during continuous administration of a medical solution, there are times when the flow rate varies due to textural variation at the medical-solution administration site such as carcinoma in a biological object. However, when the medical solution is administered with the above-mentioned osmotic pump, such variation in the flow rate goes unnoticed. As a result, there are times when a definite quantity of the medical solution cannot be administered within a predetermined time period.

The present invention has been made in view of the above, and it is an object of the present invention to provide a medical-solution administration device that enables administration of a definite quantity of a medical solution within a predetermined time period.

### MEANS FOR SOLVING PROBLEM

To solve the problem described above and achieve the object, a medical-solution administration device according to the present invention includes a medical-solution reservoir that is filled with at least a medical solution and that feeds the medical solution to a medical solution administration side; a pump that discharges to the medical-solution reservoir a driving liquid that is used in pushing the medical solution filled in the medical-solution reservoir; and a driving liquid reservoir that is filled with the driving liquid, wherein the medical-solution reservoir forms a pipe in which the driving liquid discharged by the pump enters from an opening on one end so that the medical solution is pushed to the medical solution administration side from an opening on other end of the pipe, and a marker is placed between the driving liquid and the medical solution, the marker being used in detecting a pushed quantity of the medical solution pushed by the driving liquid.

In the medical-solution administration device according to the present invention as set forth in the invention described above, the marker is a colored liquid, and the pipe is transparent.

In the medical-solution administration device according to the present invention as set forth in the invention described above, the marker is provided in the driving liquid.

In the medical-solution administration device according to the present invention as set forth in the invention described above, the marker is a driving liquid made to contain a dye.

In the medical-solution administration device according to the present invention as set forth in the invention described above, the marker is a magnetic marker.

In the medical-solution administration device according to the present invention as set forth in the invention described above, between the driving liquid and the medical solution is filled an isolating fluid including oil that prevents mixing of the driving liquid and the medical solution.

In the medical-solution administration device according to the present invention as set forth in the invention described above, the pipe is folded.

In the medical-solution administration device according to the present invention as set forth in the invention described above, the pipe is wound in a coil shape.

In the medical-solution administration device according to the present invention as set forth in the invention described above, the pipe has a uniform wound diameter.

In the medical-solution administration device according to the present invention as set forth in the invention described above, the driving liquid reservoir is disposed inside an internal space formed by the pipe wound in a coil shape.

In the medical-solution administration device according to the present invention as set forth in the invention described above, the pump is a variable flow rate pump.

In the medical-solution administration device according to the present invention as set forth in the invention described above, the variable flow rate pump is an electro-osmotic flow pump.

In the medical-solution administration device according to the present invention as set forth in the invention described above, the medical-solution reservoir includes a scale that indicates an amount of movement of the marker.

In the medical-solution administration device according to the present invention as set forth in the invention described above, a sensor that detects movement of the marker and a control unit that, based on a detection result of the sensor, controls the pump for controlling the pushed quantity of the medical solution are further included.

In the medical-solution administration device according to the present invention as set forth in the invention described above, a memory unit that stores therein a temporal profile of medical solution administration is further included, and the control unit controls a medical-solution-administration quantity per unit time corresponding to the temporal profile.

In the medical-solution administration device according to the present invention as set forth in the invention described above, the sensor is a line image sensor.

In the medical-solution administration device according to the present invention as set forth in the invention described above, the sensor is a magnetic line sensor.

In the medical-solution administration device according to the present invention as set forth in the invention described above, a biological information obtaining unit that obtains biological information of a target for administering the medical solution and a control unit that, based on an obtaining result of the biological information obtaining unit, controls a pushing quantity from the pump are further included.

In the medical-solution administration device according to the present invention as set forth in the invention described above, a memory unit in which a relation between the biological information and the medical-solution-administration quantity per unit time is stored is further included, and based on the relation stored in the memory unit, the control unit obtains a medical-solution-administration quantity per unit time corresponding to biological information obtained by the biological information obtaining unit and controls the pushing quantity from the pump in such a way that the pushing quantity is equal to the medical-solution-administration quantity per unit time.

In the medical-solution administration device according to the present invention as set forth in the invention described above, a sensor that detects movement of the marker and a control unit that, based on a detection result of the sensor, performs feedback control to maintain the medical-solution-administration quantity per unit time are further included.

In the medical-solution administration device according to the present invention as set forth in the invention described above, a memory unit that stores therein a temporal profile of medical solution administration is further included, and based on the biological information obtained by the biological information obtaining unit, the control unit controls a medical-solution-administration quantity per unit time corresponding to the temporal profile.

In the medical-solution administration device according to the present invention as set forth in the invention described above, the control unit controls a medical-solution-administration start time according to the temporal profile based on the biological information obtained by the biological information obtaining unit.

In the medical-solution administration device according to the present invention as set forth in the invention described above, a control unit that controls a pushing quantity from the pump is further included, and the control unit has an initial setting mode used in discharging the medical solution at a high velocity while delivering the medical solution for first time.

In the medical-solution administration device according to the present invention as set forth in the invention described above, the driving liquid reservoir and the medical-solution reservoir are detachable and, in an assembled condition, implantable in a human body.

In the medical-solution administration device according to the present invention as set forth in the invention described above, a retainer that retains the medical-solution administration device to outside of a biological object and a catheter that guides the medical solution discharged from the medical-solution reservoir to an affected area inside a biological object are further included, and the medical-solution administration device is portable.

In the medical-solution administration device according to the present invention as set forth in the invention described above, a maximum quantity of the driving liquid delivered from the driving liquid reservoir is smaller than a medical solution quantity filled in the medical-solution reservoir.

In the medical-solution administration device according to the present invention as set forth in the invention described above, a driving liquid quantity filled in the driving liquid reservoir is smaller than a medical solution quantity filled in the medical-solution reservoir.

In the medical-solution administration device according to the present invention as set forth in the invention described above, from a driving liquid quantity filled in the driving liquid reservoir, a liquid quantity suppliable to the pump is smaller than a medical solution quantity filled in the medical-solution reservoir.

In the medical-solution administration device according to the present invention as set forth in the invention described above, a spacer that is disposed flowably between the medical solution and the driving liquid and a blocking member that blocks flowing of the spacer in a flow path that is beyond a delivery side end of the medical-solution reservoir and outside of a biological object are further included.

In the medical-solution administration device according to the present invention as set forth in the invention described above, a magnetic bead that is disposed between the medical solution and the driving liquid and a holding unit that holds the magnetic bead in a flow path that is beyond a delivery side end of the medical-solution reservoir and outside of a biological object are further included.

In the medical-solution administration device according to the present invention as set forth in the invention described above, the holding unit includes a blocking member that blocks flowing of the magnetic bead.

In the medical-solution administration device according to the present invention as set forth in the invention described above, a detecting unit that detects one or more of a driving liquid remaining quantity in the driving liquid reservoir, a flow rate of the driving liquid from the pump, and a flow rate of the medical solution from the medical-solution reservoir; and a control unit that, based on a detection quantity of the detecting unit, performs control to stop the pump from discharging the driving liquid so that the driving liquid does not flow to a tip side of a flow path that is beyond a delivery side end of the medical-solution reservoir and outside of a biological object are further included.

In the medical-solution administration device according to the present invention as set forth in the invention described above, the marker is a spacer that is disposed flowably between the medical solution and the driving liquid, a stop is disposed for blocking flowing of the spacer in the flow path beyond a delivery side end of the medical-solution reservoir and outside of a biological object, and a driving liquid quantity delivered from the driving liquid reservoir is smaller than a medical solution quantity filled in the medical-solution reservoir.

### EFFECT OF THE INVENTION

According to an aspect of the present invention, a medical-solution reservoir forms a pipe in which a driving liquid pumped by a pump enters from an opening on one end so that a medical solution is pushed to a medical-solution administration side from an opening on the other end of the pipe. Between the driving liquid and the medical solution is placed a marker for detecting a pushed quantity of the medical solution pushed by the driving liquid. As a result, it becomes possible to know the state of medical-solution administration and administer a definite quantity of the medical solution that needs to be administered within a predetermined time period.

### BRIEF DESCRIPTION OF DRAWINGS

FIG. 1 is a schematic diagram showing a situation when a medical-solution administration device according to a first embodiment of the present invention is applied to a human body.
FIG. 2 is a schematic diagram showing a configuration of the medical-solution administration device illustrated in FIG. 1.
FIG. 3 is a diagram showing a positional relation between a driving liquid, a marker, and a medical solution.
FIG. 4 is a diagram showing a positional relation between a driving liquid containing a dye and the medical solution.
FIG. 5 is a perspective view showing a configuration in which a driving liquid reservoir is disposed inside the internal space of a medical-solution reservoir.
FIG. 6 is a longitudinal sectional view showing a configuration in which the driving liquid reservoir is disposed inside the internal space of the medical-solution reservoir.
FIG. 7 is a schematic diagram showing a configuration of a medical-solution administration device according to a second embodiment of the present invention.
FIG. 8 is a diagram showing an example of flow rate control with respect to a temporal profile representing a constant quantity of unit time flow rate.
FIG. 9 is a diagram showing an example of flow rate control with respect to a temporal profile representing a variable quantity of unit time flow rate.
FIG. 10 is a diagram showing a modification example of the medical-solution reservoir.
FIG. 11 is a longitudinal sectional view showing a configuration in which a line sensor is disposed inside the internal space of the medical-solution reservoir.
FIG. 12 is a schematic diagram showing a configuration of a medical-solution administration device according to a third embodiment of the present invention.
FIG. 13 is a diagram showing a positional relation between the driving liquid, a magnetic marker, and the medical solution.
FIG. 14 is a schematic diagram showing a configuration of a medical-solution administration device according to a fourth embodiment of the present invention.
FIG. 15 is a diagram showing an example of biological-information/flow rate relation stored in a memory unit.
FIG. 16 is a diagram showing an example of a relation between a temporal profile and biological information stored in the memory unit.
FIG. 17 is a diagram showing an example of a relation between a temporal profile and biological information stored in the memory unit.
FIG. 18 is a perspective view showing a configuration in which the driving liquid reservoir is disposed inside the internal space of the medical-solution reservoir.
FIG. 19 is a longitudinal sectional view showing a configuration in which the driving liquid reservoir is disposed inside the internal space of the medical-solution reservoir.
FIG. 20 is a longitudinal sectional view showing a configuration in which a magnetic line sensor is disposed inside the internal space of the medical-solution reservoir.
FIG. 21 is a schematic diagram showing a configuration of a medical-solution administration device according to a fifth embodiment of the present invention.
FIG. 22 is an explanatory diagram showing a condition when the driving liquid remains inside the driving liquid reservoir without being discharged.

### EXPLANATIONS OF LETTERS OR NUMERALS

1 Human body
2 Affected area
3 Medical-solution administration device
4 Retainer
5 Catheter
6 Feed tube
10 Medical solution reservoir
10a Stop
11 Marker
12 Electro-osmotic flow pump
13 Driving liquid reservoir
14 Flow rate setting unit
15 Operating unit
16 Scale
20, 30 Driving liquid
21 Medical solution
22a, 22b, 32 Oil
23a to 23d, 33a, 33b Air
40, 40a Line sensor
41 Control unit
41a Flow-rate-variation detecting unit
41b Pump control unit
41c Biological information detecting unit
42 Input-output unit
43 Memory unit
43a Unit-time flow-rate memory unit
106 Connecting unit
111 Magnetic marker
124 Biological information sensor
140 Magnetic line sensor
143a Biological-information/flow-rate relation memory unit

### BEST MODE(S) FOR CARRYING OUT THE INVENTION

Exemplary embodiments of a medical-solution administration device according to the present invention will be described below in detail with reference to the accompanying drawings. The present invention is not limited to the embodiments described below.

### First Embodiment

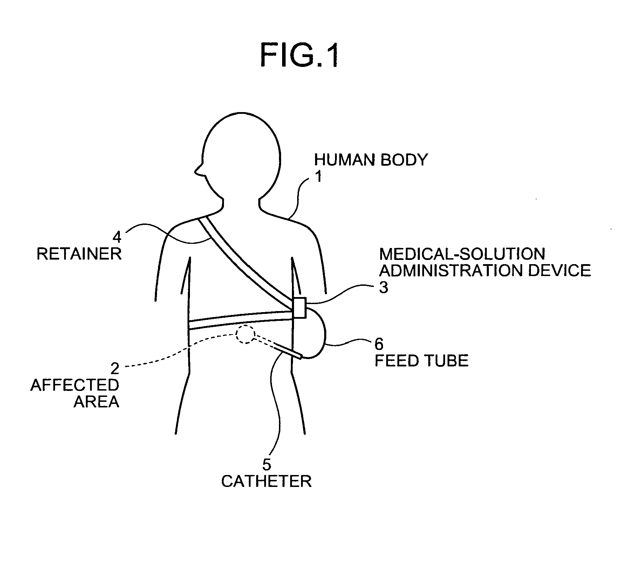

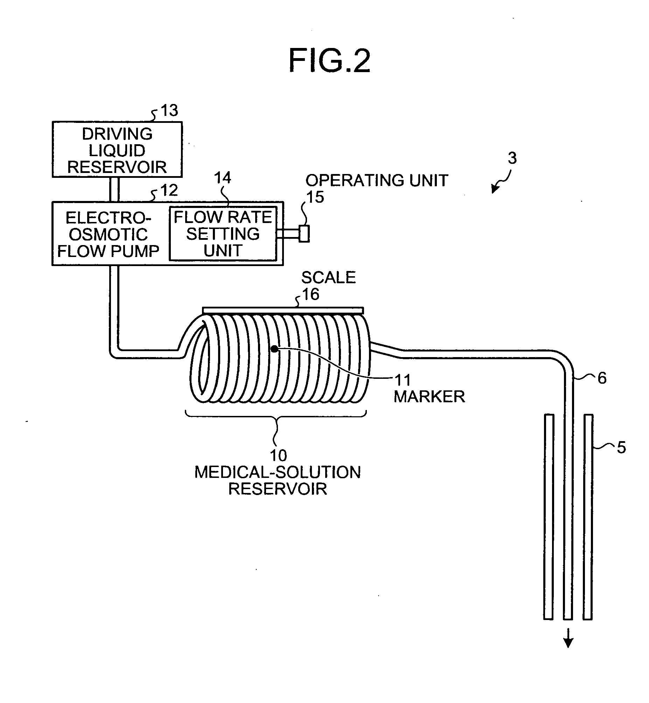

FIG. 1 is a schematic diagram showing a situation when a medical-solution administration device according to a first embodiment of the present invention is applied to a human body. FIG. 2 is a schematic diagram showing a configuration of the medical-solution administration device illustrated in FIG. 1. A medical-solution administration device 3 discharges and administers, to an affected area 2 such as carcinoma inside a human body 1 as the biological object, a medical solution such as an anticancer drug of fluorouracil (5-FU) of about tens of ml in a continuous and focused manner over a long time period of about one week. The medical solution discharged continuously from the medical-solution administration device 3 is administered to the affected area 2 via a feed tube 6 that is inserted in a catheter 5 connecting between the outside of the human body 1 and the affected area 2. The medical-solution administration device 3 is portably retained on the human body 1 with a retainer 4 such as a belt. The portability of the medical-solution administration device 3 frees the human body 1 from any restrictions on the movements and enables extended and continuous administration of the medical solution to the affected area 2.

As illustrated in FIG. 2, the medical-solution administration device 3 includes a medical-solution reservoir 10 filled with a medical solution, a driving liquid reservoir 13 filled with a driving liquid that is used in pushing the medical solution, and an electro-osmotic flow pump 12 that is disposed between the driving liquid reservoir 13 and the medical-solution reservoir 10 and that pumps the driving liquid used in pushing the medical solution. Meanwhile, it is preferable to use ultrapure water as the driving water. The operating principle of the electro-osmotic is as follows. In an electro-osmotic material (porous body), the surface inside fine pores is charged negatively and in the vicinity of the surface occurs an excess of positive ions. The positive ions become mobile when subject to force due to an electric field applied from outside. That causes a flow in the driving liquid and the driving liquid gets discharged. Thus, by varying the electrical field intensity, it is possible to change the discharged quantity of the driving liquid. The electro-osmotic flow pump 12 includes a flow rate setting unit 14 and, corresponding to the flow rate set in the flow rate setting unit 14, generates an electric field for varying the discharging flow rate of the driving liquid. An operating unit 15 that is configured using a dial or the like is disposed for specifying the flow rate to be set in the flow rate setting unit 14.

The medical-solution reservoir 10 is a thin pipe densely-wound into a coil shape of uniform diameter and is filled with a medical solution such as an anticancer drug. The driving liquid enters that pipe from the side of the electro-osmotic flow pump 12. Thus, by getting pushed by the driving liquid, the medical solution is discharged from the medical-solution reservoir 10 in an indirect manner.

The medical-solution reservoir 10 is made of a transparent material. Moreover, a colored marker 11 made of red food coloring or the like is placed in at least some portion such as the tip portion of the driving liquid. When the medical solution is discharged, the marker 11 moves over and it is possible to keep track of the amount of movement or the condition of movement of the moving marker 11.

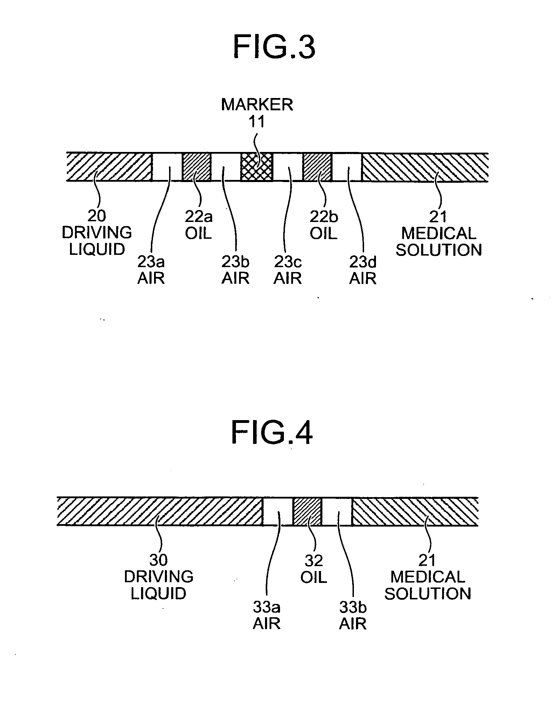

Regarding the proximity of the boundary between the driving liquid and the medical solution, as illustrated in FIG. 3, the marker 11 is placed between a driving liquid 20 and a medical solution 21. Besides, between the driving liquid 20 and the marker 11 is filled oil 22a. Between the driving liquid 20 and the oil 22a is filled air 23a and between the oil 22a and the medical solution 21 is filled air 23b. Similarly, between the medical solution 21 and the marker 11 is filled oil 22b. Between the medical solution 21 and the oil 22b is filled air 23d and between the oil 22b and the marker 11 is filled air 23c. Such a configuration enables discharging of the medical solution 21 while preventing the mixing of the driving liquid 20 and the medical solution 21.

As illustrated in FIG. 2, on the outside of the medical-solution reservoir 10 and along the axial direction of the coil is disposed a scale 16 made of a transparent material. Moreover, it is desirable that the scale 16 can be pasted on the medical-solution reservoir 10 in a seal shape. The scale 16 can be used to measure the amount of movement of the marker 11 so that the variation in the flow rate of the medical solution is known. Alternatively, instead of disposing the scale 16, it is also possible to directly write a scale on the surface of the medical-solution reservoir 10. Particularly, since the medical-solution reservoir 10 is coiled with a uniform diameter and a uniform pitch, a straight line can be marked along the axial direction for checking the variation in the time taken by the marker 11 to make one round. That makes it possible to know the variation in the flow rate of the medical solution. Even if the scale 16 is not disposed, the variation in the flow rate of the medical solution can be found out by checking the variation in the time taken by the marker 11 to make one round at almost the same position.

If any variation in the flow rate of the medical solution is known, then the operator can operate the operating unit 15 to change the flow rate setting value of the flow rate setting unit 14 and make sure that the flow rate is reset to that prior to the occurrence of variation. That eventually makes it possible to administer the total dose of the medical solution that needs to be administered within the administration period. Particularly, in a conventional medical-solution administration device, variations in the flow rate of a medical solution within the administration period are not known. Thus, even if, after the completion of the administration period, it is realized that the required total dose of the medical solution was not administered; it is not possible to correct the inadequate administration of the medical solution. In contrast, in the present embodiment, it is possible to know the variation in the flow rate of the medical solution during the administration period. Hence, corrections can be made to the administration of the medical solution during the administration period and the total required dose of the medical solution can be administered within the administration period. In this way, it is possible to perform adequate administration of the medical solution.

Besides, instead of disposing the marker 11 as in the case of the above-mentioned first embodiment, as illustrated in FIG. 4, a driving liquid 30 itself can be made to contain a dye and changes in the position of the tip portion of the driving liquid 30 can be checked to know the variation in the flow rate of the medical solution. In FIG. 4, to prevent the mixing of the driving liquid 30 and the medical solution 21, oil 32 is filled therebetween. Moreover, between the oil 32 and the driving liquid 30 is filled air 33a and between the oil 32 and the medical solution 21 is filled air 33b.

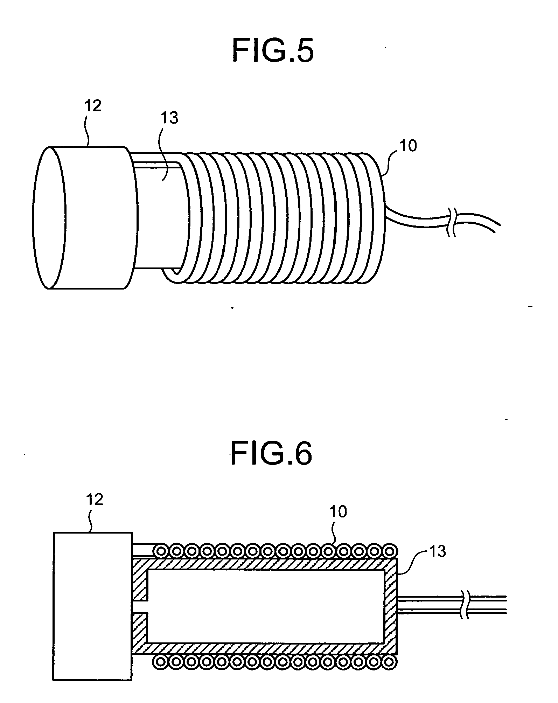

As illustrated in FIGS. 5 and 6, the driving liquid reservoir 13 can be disposed inside the internal space formed by the coil-shaped pipe of the medical-solution reservoir 10. In such cases, since the internal space is cylindrical in shape, it is desirable to manufacture the driving liquid reservoir 13 from, for example, a columnar tube of fluorine contained resin. Moreover, it is desirable to dispose the electro-osmotic flow pump 12 adjacent to the driving liquid reservoir 13 and the medical-solution reservoir 10. By disposing the driving liquid reservoir 13 inside the internal space of the medical-solution reservoir 10, the medical-solution administration device 3 can be reduced in size and can have an enhanced portability. The downsized medical-solution administration device 3 can also be implanted in the human body so that the person being administered the medical solution can move around more freely.

### Second Embodiment

Given below is the description of a second embodiment according to the present invention. In the above-mentioned first embodiment, the variation in the flow rate of the medical solution is known on the basis of the amount of movement of the marker 11 and, when there is variation in the flow rate of the medical solution, the operator operates the operating unit 15 to correct the flow rate of the medical solution. In comparison, in the second embodiment, a line sensor is disposed to detect the amount of movement of the marker 11 and the flow rate of the medical solution is corrected in an automatic manner based on the detection result of the line sensor.

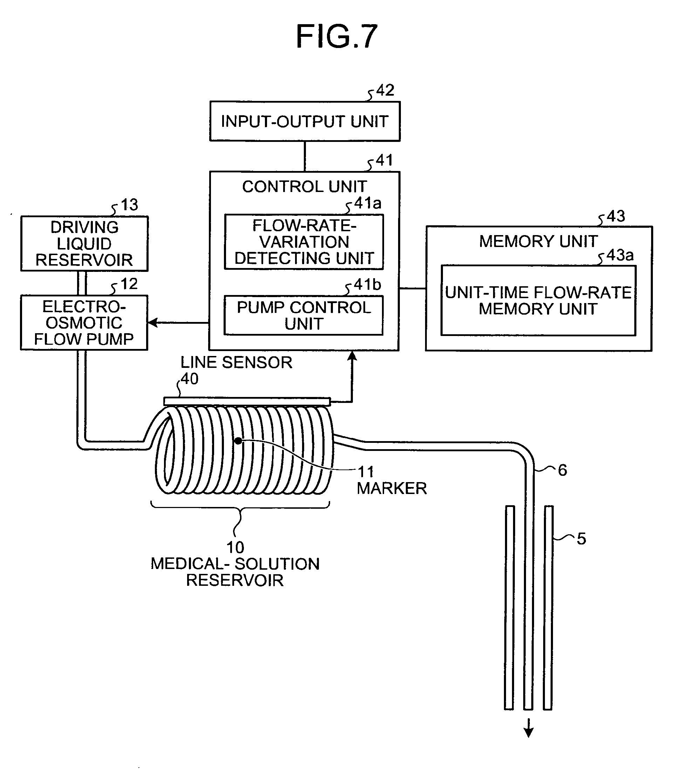

FIG. 7 is a schematic diagram showing a configuration of the medical-solution administration device according to the second embodiment of the present invention. The medical-solution administration device illustrated in FIG. 7 includes a line sensor 40 that detects the amount of movement of the marker 11 disposed inside the medical-solution reservoir 10; a control unit 41 that, based on the detection result of the line sensor 40, at least controls the flow rate of the driving liquid pumped from the electro-osmotic flow pump 12; an input-output unit 42 that is used in issuing a variety of input instructions or settings with respect to the medical-solution administration device and performing output such as display; and a memory unit 43. Apart from that, the configuration of the medical-solution administration device is identical to that according to the first embodiment. Hence, the identical constituent elements are referred by the same reference numerals.

The line sensor 40 is a line image sensor that is disposed along the axial direction of and on the surface of the medical-solution reservoir 10. The control unit 41 includes a flow-rate-variation detecting unit 41a that detects variation in the flow rate by performing image processing on the passing of the marker 11. The control unit 41 also includes a pump control unit 41b that, on the basis of the variation in the flow rate detected by the flow-rate-variation detecting unit 41a, controls the flow rate of the driving liquid pumped from the electro-osmotic flow pump 12, that is, controls the flow rate of the medical solution. More particularly, the pump control unit 41b performs feedback control to ensure that the medical solution always flows in a constant quantity per unit time. Because of the feedback control, the flow rate of the medical solution can be corrected in a reliable manner.

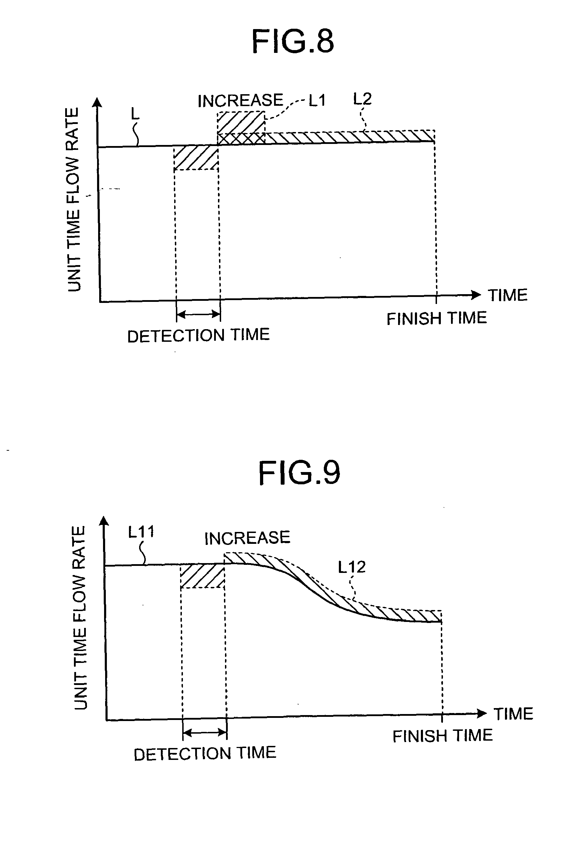

Besides, the control unit 41 can also be configured to perform control so that the total dose of the medical solution is reliably administered within the administration period. In that case, the control unit 41 performs control based on the temporal profile of a unit time flow rate that is stored in a unit-time flow-rate memory unit 43a of the memory unit 43. For example, as illustrated in FIG. 8, consider a case of administering the medical solution corresponding to a straight line L that represents a constant quantity of the unit time flow rate within the administration period. In that case, if the unit time flow rate drops at a detection time of the line sensor 40; then, to correct the dropped flow rate, the unit time flow rate can be increased at the subsequent detection time in order to compensate for the decreased quantity of the medical solution and the flow rate can be controlled with the straight line L of the increased unit time flow rate value as the target value. Meanwhile, since the ultimate requirement is to administer the total dose of the medical solution before the administration period is over; it is also possible to control the flow rate, during the period from the point of time at which the drop was detected to the finish time of the administration period, with a straight line L2 of a unit time flow rate value obtained by equalization of the decreased quantity as the target value.

As illustrated in FIG. 9, when the temporal profile is represented by a curve line L11; then, for example, with a curve line L12 as the target value that increases after equalization during the period from the point of time at which the decrease was detected to the finish time of the administration period, the feedback control is performed on the quantity of the medical solution decreased in the detection period.

In the second embodiment, since the control unit 41 performs the feedback control of the flow rate on the basis of the amount of movement of the marker 11 detected by the line sensor 40, it is possible to reliably administer the total required dose of the medical solution within the administration period.

Meanwhile, in the first embodiment, the flow rate is changed using the operating unit 15. Instead, it is also possible to separately dispose an electro-osmotic flow pump that, in response to variation in the flow rate, pumps the driving liquid at a desired flow rate.

Moreover, when the medical-solution administration device is set on the human body 1, the feed tube 6 does not generally contain the medical solution. Hence, an initial setting is performed to fill the medical solution in the feed tube 6. If the flow rate at the time of performing the initial setting is same as the flow rate at the time of administering the medical solution, then it takes a substantial amount of time to fill the medical solution in the feed tube 6. Thus, while filling the medical solution in the feed tube 6 at the time of the initial setting, the medical solution is discharged with a high flow rate. For example, in the first embodiment, such discharging can be controlled by operating the operating unit 15. Moreover, even in the case of replacing the electro-osmotic flow pump 12, it can be replaced with a pump that can discharge the medical solution at a high flow rate at the time of initial setting. In the second embodiment, the control unit 41 ensures that the medical solution is discharged with a high flow rate at the time of initial setting.

According to the above-mentioned first and second embodiments, the medical-solution reservoir 10 is a thin pipe densely-wound into a coil shape of uniform diameter. Alternatively, the medical-solution reservoir 10 can also be a transparent pipe wound not in a circular manner but in a rectangular manner. What matters is that the medical-solution reservoir 10 is transparent so that it is possible to check the amount of movement of the marker.

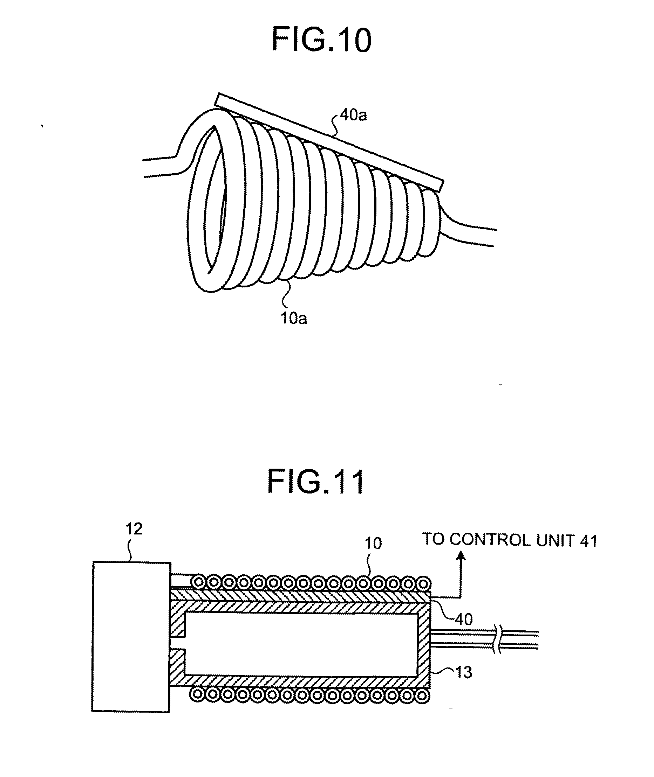

With respect to the second embodiment, as illustrated in FIG. 10, the medical-solution reservoir 10 can also be manufactured as a coil-shaped pipe having a tapering diameter. In that case, it is desirable that the coil-shaped pipe has a uniform gradient along the axial direction. That facilitates easy installation of and easy detection by a line sensor 40a. Meanwhile, the relation between the detection of the marker and the amount of movement of the marker needs to be corrected.

In the above-mentioned first and second embodiments, the marker 11 is assumed to be made of red food coloring. Alternatively, it is also possible to use a colored plastic ball. What matters is that a person is able to visually confirm the marker 11 in the first embodiment and the line sensor 40 is able to detect the marker 11 in the second embodiment.

As described with reference to FIG. 6, the driving liquid reservoir 13 can be disposed inside the internal space of the medical-solution reservoir 10. Besides, as illustrated in FIG. 11, the line sensor 40 can also be disposed inside the internal space of the medical-solution reservoir 10. Alternatively, it is also possible to dispose only the line sensor 40 inside the internal space of the medical-solution reservoir 10.

### Third Embodiment

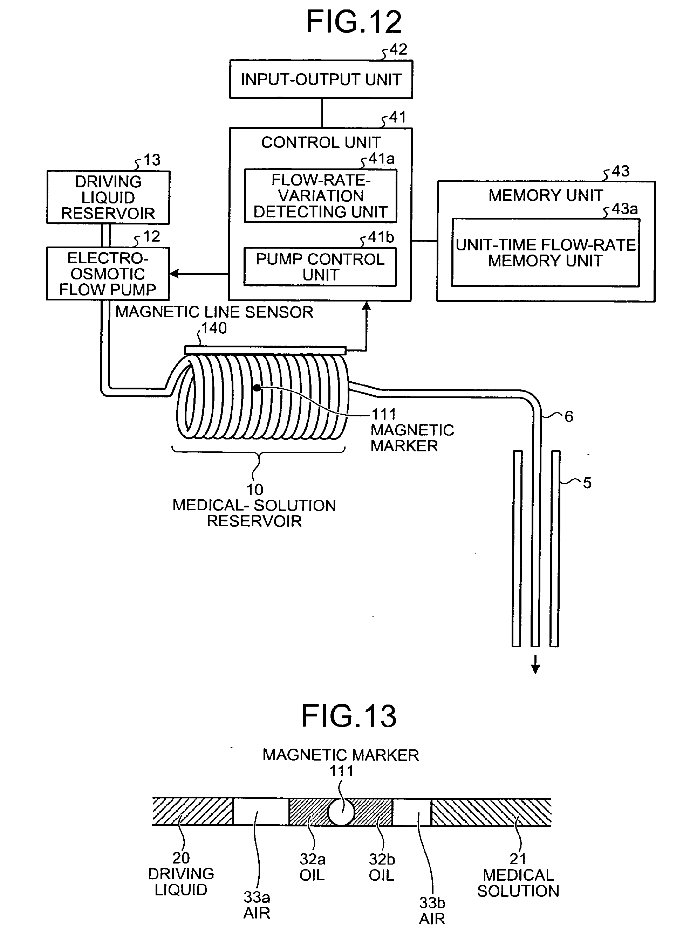

Given below is the description of a third embodiment according to the present invention. In the above-mentioned second embodiment are used the colored marker 11 and the line sensor 40 that detects the amount of movement of the marker 11. In the third embodiment, the marker 11 and the line sensor 40 are respectively replaced with a magnetic marker 111 and a magnetic line sensor 140 as illustrated in FIG. 12. Apart from that, the configuration is identical to the medical-solution administration device illustrated in FIG. 7. Hence, the identical constituent elements are referred by the same reference numerals.

As illustrated in FIG. 13, on both sides of the magnetic marker 11 is filled oil 32a and oil 32b, and on the outside of the oil 32a and the oil 32a is respectively filled the air 33a and the air 33b. The driving liquid 20 and the medical solution 21 remain filled outside of the air 33a and the air 33b, respectively. The magnetic line sensor 140 is disposed along the axial direction of and on the external surface of the medical-solution reservoir 10. When the magnetic marker 111 moves over due to the discharging of the medical solution, it is possible to keep track of the amount of movement or the condition of movement of the magnetic marker 111 using the magnetic line sensor 140.

### Fourth Embodiment

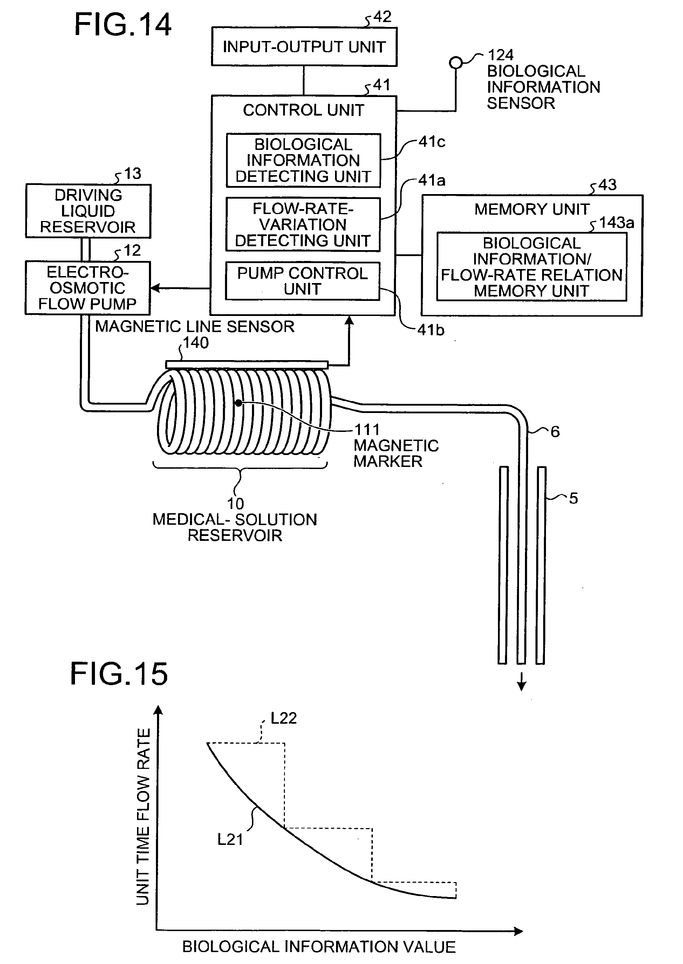

Given below is the description of a fourth embodiment according to the present invention. According to the fourth embodiment, as illustrated in FIG. 14, a biological information sensor 124 is newly disposed in the medical-solution administration device described in the third embodiment with reference to FIG. 12. The biological information sensor 124 detects biological information of the human body 1, which is the biological object being administered the medical solution. The biological information includes information on, for example, brain waves, blood components or blood constitutes, and cardiac rate that are related to the condition of the affected area due to the administered medical solution.

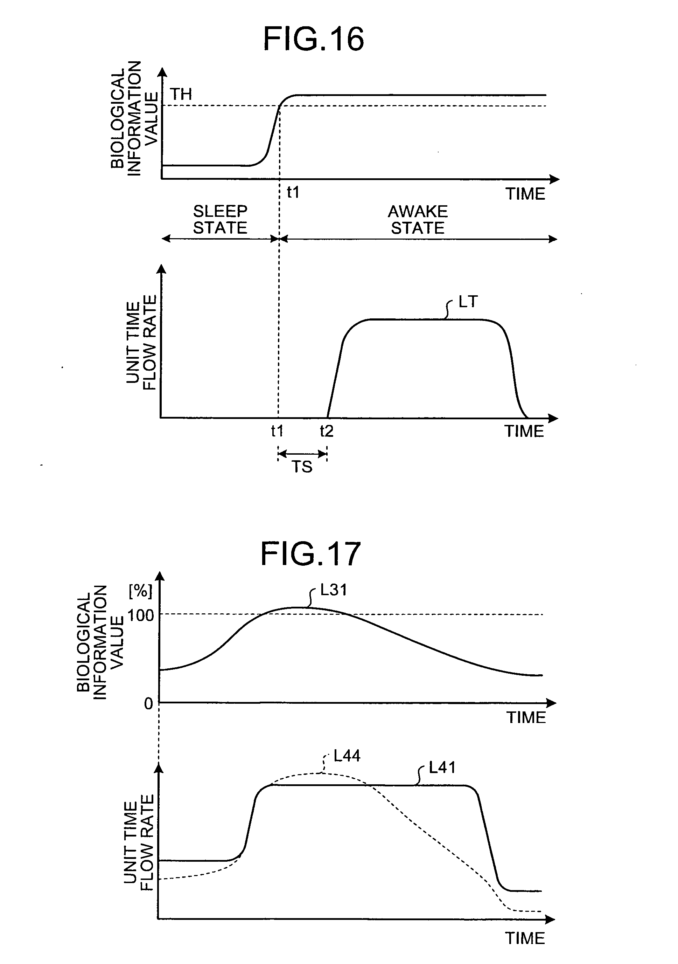

In the fourth embodiment, the memory unit 43 includes a biological-information/flow-rate relation memory unit 143a in place of the unit-time flow-rate memory unit 43a. In the biological-information/flow-rate relation memory unit 143a is stored, as illustrated in FIG. 15, the relation between a biological information value detected by the biological information sensor 124 and the unit time flow rate of the medical solution. Meanwhile, the biological-information/flow-rate relation memory unit 143a can be configured to store the temporal profile that is the time variation of the unit time flow rate as illustrated in the lower graphs in FIGS. 16 and 17.

In the fourth embodiment, the control unit 41 also includes a biological information detecting unit 41c that detects the biological information value based on the value detected by the biological information sensor 124. The flow-rate-variation detecting unit 41a in the control unit 41 detects variation in the flow rate of the medical solution when the magnetic line sensor 140 detects the passing of the magnetic marker 111. The pump control unit 41b in the control unit 41 obtains the unit time flow rate based on the biological information value detected by the biological information detecting unit 41c and the biological-information/flow rate relation stored in the biological-information/flow-rate relation memory unit 143a. Then, the pump control unit 41b controls the flow rate of the driving liquid pumped from the electro-osmotic flow pump 12, that is, controls the flow rate of the medical solution in such a way that the variation in the flow rate detected by the flow-rate-variation detecting unit 41a becomes equal to the obtained unit time flow rate.

The control unit 41 performs pump control based on, for example, the biological-information/flow rate relation illustrated in FIG. 15. With reference to FIG. 15, the control unit 41 performs feedback control based on the detection result of the flow-rate-variation detecting unit 41a so that the medical solution is administered at the unit time flow rate corresponding to the detected biological information value along a characteristic curve L21 illustrated by a solid line. Alternatively, as illustrated by a dashed curve line L22 in FIG. 15, it is also possible to perform control so that the unit time flow rate becomes discrete values corresponding to the biological information value. Herein, based on the detection result of the flow-rate-variation detecting unit 41a; the control unit 41 attempts to perform feedback control with the biological-information/flow rate relation as the target value. Alternatively, instead of performing feedback control, the flow rate of the medical solution can be controlled in a feedforward manner on the basis of the detection result of the biological information detecting unit 41c and the biological-information/flow rate relation.

Since the control unit 41 controls the flow rate of the medical solution using the biological information, the medical solution can be adequately administered according to the condition of the biological object while ensuring that there is no adverse effect on the biological object. That enables achieving enhancement in the impact of the medical-solution administration according to the condition of the biological object.

As a specific example of the relation between biological information and medical-solution administration management, a sensor that detects the value of deviation enzymes such as AST (aspartate aminotransferase) and ALT (alanine aminotransferase) in the liver can be disposed as the biological information sensor 124. In that case, if the detected biological information value shows a rise; then, based on the biological-information/flow rate relation corresponding to an assessment result indicating toxicity effect, the control unit 41 can perform control to reduce the unit time flow rate during administration of the medical solution.

Alternatively, a sensor that detects a serum creatinine value indicating a type of waste material present in the blood can be disposed as the biological information sensor 124. In that case, if the detected serum creatinine value shows a rise; then, based on the biological-information/flow rate relation corresponding to an estimation result indicating impaired liver functioning, the control unit 41 can perform control to reduce the unit time flow rate during administration of the medical solution.

Still alternatively, in the case of performing chemotherapy on prostate cancer; the biological information sensor 124 can be configured to measure the level of PSA (prostate specific antigen), which is a type of prostate-specific protein, in the blood. Then, based on that blood level value, the control unit 41 controls the unit time flow rate during administration of the medical solution on the basis of the biological-information/flow rate relation corresponding to the effectiveness of the treatment.

Still alternatively, the temporal profile of the unit time flow rate can be stored in the biological-information/flow rate relation and the control unit 41 can be configured to control the flow rate using that temporal profile. For example, as illustrated in the lower graph in FIG. 16, a temporal profile LT of the unit time flow rate of the medical solution is stored in addition to the biological-information/flow rate relation. Then, assume that the biological information sensor 124 is configured to perform brain wave detection or pulse detection for detecting whether the human body 1 is asleep or awake and assume that, at a time t1, the detection result indicates the transition from sleep to wakefulness in the human body 1. Then, the control unit 41 controls the flow rate of the medical solution to ensure that the unit time flow rate is equal to that indicated by the temporal profile LT at a time t2, which comes after the time t1 by a time interval TS. Naturally, depending on the medical solution, it is also possible to control the flow rate of that medical solution corresponding to the temporal profile LT immediately after the time t1. By performing such control, it becomes possible to perform accurate chronopharmacological chronotherapeutics according to the condition of the biological object and enhance the impact of the medical-solution administration.

Alternatively, the control unit 41 can be configured to correct, according to a curve line L31 representing the detection result of the biological information value, a temporal profile L41 illustrated in the lower graph in FIG. 17 to an optimal temporal profile L44 in response to the condition of the biological object, and then control the flow rate of the medical solution based on the temporal profile L44. Regarding the temporal profile L41, according to the largeness of the changing biological information value (L31); the correction percentage of the temporal profile L41 is varied, in other words, positive and negative weighting is performed, and the temporal profile L41 is corrected to the temporal profile L44. Thus, it becomes possible to adequately administer the medical solution according to the biological information value.

Meanwhile, it is desirable that the biological information sensor 124 performs direct and real-time detection of the biological information of the human body 1. However, indirect detection on the outside of the body is also possible as long as the biological information is detected in almost real time.

The control unit 41 can also be configured to display information such as the above-mentioned biological information value, the temporal variation in the biological information value, and the flow rate control status on a display unit (not illustrated) disposed in the input-output unit 42. Moreover, the electro-osmotic flow pump 12 can be equipped with an offline flow rate setting unit (not illustrated) that is not kept under the control of the control unit 41. In that case, the operations are performed manually with an operating unit configured using a dial or the like.

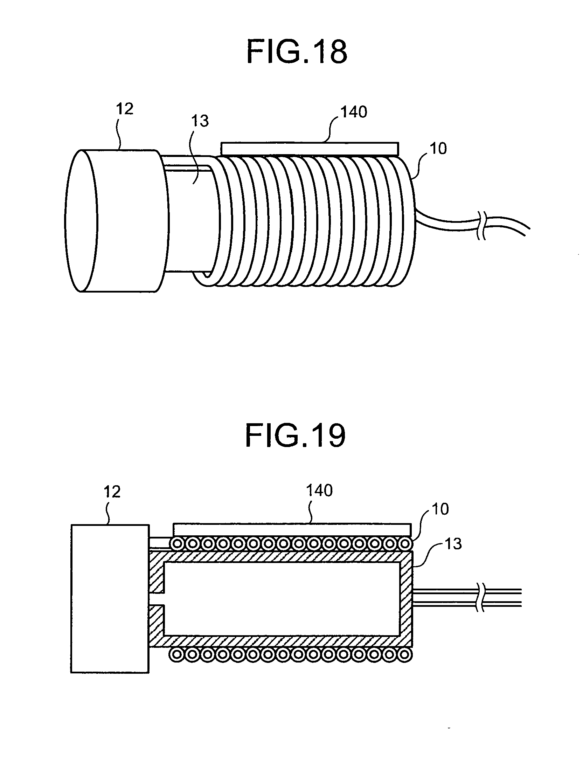

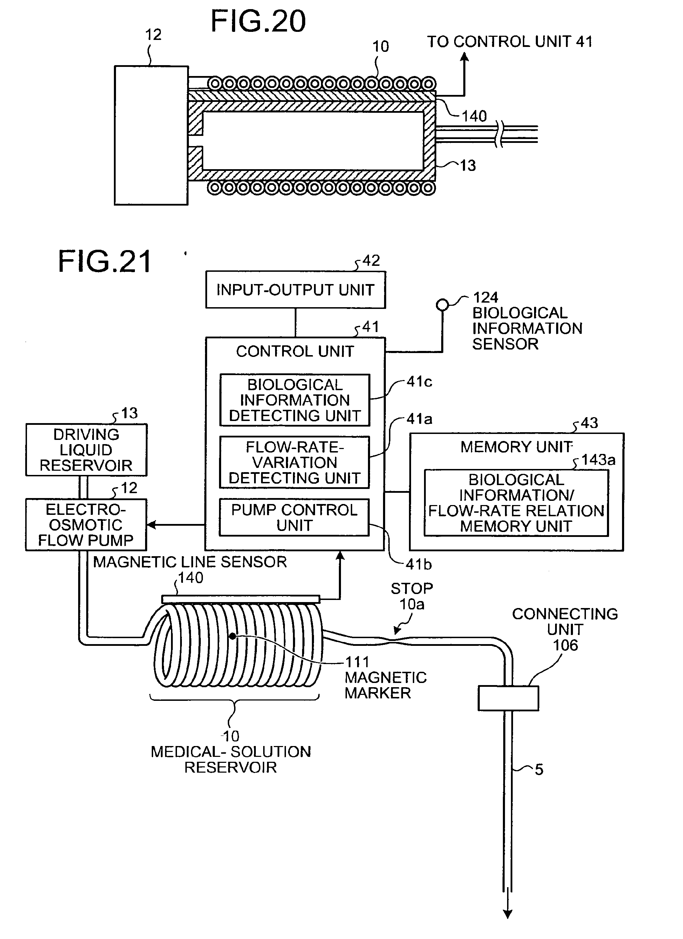

As illustrated in FIGS. 18 and 19, the driving liquid reservoir 13 can be disposed inside the internal space of the medical-solution reservoir 10. Moreover, as illustrated in FIG. 20, the magnetic line sensor 140 can also be disposed inside the internal space of the medical-solution reservoir 10. Alternatively, it is also possible to dispose only the magnetic line sensor 140 inside the internal space of the medical-solution reservoir 10.

In the present embodiment, the magnetic marker 11 is disposed inside the medical-solution reservoir 10 and the variation in the flow rate of the medical solution is detected by the magnetic line sensor 20 based on the amount of movement of the magnetic marker 11. Alternatively, as described in the first embodiment, the line sensor 40 can also be used as the line image sensor for detecting the colored marker 11 inside the medical-solution reservoir 10 and the flow rate can be controlled based on the detection result.

### Fifth Embodiment

Given below is the description of a fifth embodiment according to the present invention. In the fifth embodiment, the configuration illustrated in FIG. 21 corresponds to that in the fourth embodiment. However, the quantity of the driving liquid discharged from the driving liquid reservoir 13 via the electro-osmotic flow pump 12 is set to be smaller than the quantity of the medical solution filled in the medical-solution reservoir 10. Thus, the driving liquid runs short before the rear end portion of the medical solution being discharged reaches the delivery side fore-end of the medical-solution reservoir 10. For that reason, the driving liquid is no more discharged and the medical solution reliably stops from flowing beyond the delivery side fore-end of the medical-solution reservoir 10. That enables forestalling of the driving liquid from being administered.into the human body 1.

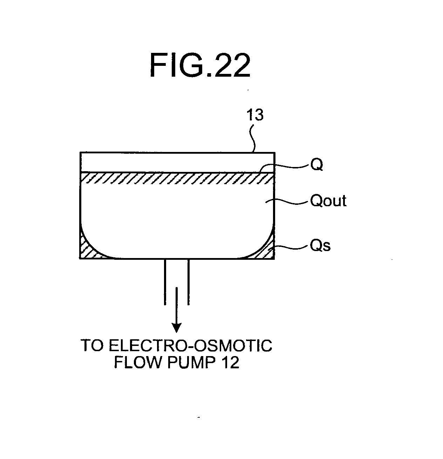

Herein, it is desirable that the quantity of the driving liquid filled in the driving liquid reservoir 13 is smaller than the quantity of the medical solution filled in the medical-solution reservoir 10. Moreover, as illustrated in FIG. 22, from a driving liquid quantity Q filled in the driving liquid reservoir 13; it is desirable that a liquid quantity Qout that can be supplied to the electro-osmotic flow pump 12 is kept smaller than the quantity of the medical solution filled in the medical-solution reservoir 10. As illustrated in FIG. 22, from the driving liquid quantity Q filled in the driving liquid reservoir 13; a liquid quantity Qs is present in actuality at the corners inside the tank of the driving liquid reservoir 13. Thus, the liquid quantity Qout that is obtained by subtracting the liquid quantity Qs from the driving liquid quantity Q and that can be practically supplied to the electro-osmotic flow pump 12 needs to be kept smaller than the quantity of the medical solution filled in the medical-solution reservoir 10.

In the fifth embodiment, as illustrated in FIG. 21, a stop 10a is disposed in the flow path beyond the delivery side fore-end of the medical-solution reservoir 10. The stop 10a acts as a blocking member for the flow path toward outside of the biological object. That is, the stop 10a traps the magnetic marker 111 placed between the driving liquid 20 and the medical solution 21 and prevents the driving liquid 20 from being discharged. That enables forestalling of the driving liquid 20 from being administered into the human body 1.

The stop 10a can also be made of a metal for trapping the magnetic marker 111 so that the driving liquid 20 can be reliably prevented from being administered.

Meanwhile, in the fifth embodiment, the magnetic marker 111 is placed between the driving liquid 20 and the medical solution 21. Alternatively, a spacer that is of a trappable size in the stop 10a and that is movable inside the flow path can be disposed between the driving liquid 20 and the medical solution 21.

Moreover, as the blocking member, the stop 10a can be replaced with a net inside the tube, or a ring that can be fit inside the tube, or a pin across the tube; because what matters is that the movable spacer such as the magnetic marker 111 is trapped at the position of the stop 10a and the driving liquid is prevented from being discharged.

In the fifth embodiment, the flow-rate-variation detecting unit 21a detects variation in the flow rate of the medical solution based on the position of the magnetic marker 111 detected by the magnetic line sensor 140. Then, based on those detection results, the pump control unit 21b controls the flow rate of the driving liquid pumped from the electro-osmotic flow pump 12. Herein, it is desirable that, based on the flow rate variation detected by the flow-rate-variation detecting unit 21a, the pump control unit 21b determines whether the driving liquid 20 has been discharged in the flow path that is beyond the delivery side fore-end of the medical-solution reservoir 10 and outside of the biological object. If the driving liquid 20 has been discharged in the flow path that is beyond the delivery side fore-end of the medical-solution reservoir 10 and outside of the biological object, then it is desirable that the pump control unit 21b performs control so that the electro-osmotic flow pump 12 stops pumping of the driving liquid 20. By performing such control, the driving liquid 20 can be reliably prevented from being administered into the human body 1.

The control for stopping the driving of the electro-osmotic flow pump 12 performed by the pump control unit 21b is not dependent only on the detection of variation in the flow rate of the medical solution. Alternatively, for example, the flow rate of the driving liquid 20 pumped from the electro-osmotic flow pump 12 can be detected and the position of the tip portion of the driving liquid 20 can be estimated based on that detection result. If it is determined that the tip portion of the driving liquid 20 has reached the flow path that is beyond the delivery side fore-end of the medical-solution reservoir 10 and outside of the biological object, then control can be performed so that the electro-osmotic flow pump 12 stops pumping of the driving liquid 20. Still alternatively, a remaining quantity sensor can be disposed to detect the quantity of the driving liquid remaining in the driving liquid reservoir 13 and if, based on the detection result of the remaining quantity sensor, it is determined that the leading end of the driving liquid has reached the flow path that is beyond the delivery side fore-end of the medical-solution reservoir 10 and outside of the biological object, then control can be performed so that the electro-osmotic flow pump 12 stops pumping of the driving liquid 20.

Meanwhile, in the fifth embodiment, the medical-solution reservoir 10 and the catheter 5 are connected via a connecting unit 106, which can be configured to include a check valve that allows the medical solution flow only from the medical-solution reservoir 10 toward the catheter 5. Hence, it becomes possible to prevent overflow of bodily fluids from the human body 1.

## Claims

1. A medical-solution administration device comprising:
a medical-solution reservoir that is filled with at least a medical solution and that feeds the medical solution to a medical solution administration side;
a pump that discharges to the medical-solution reservoir a driving liquid that is used in pushing the medical solution filled in the medical-solution reservoir; and
a driving liquid reservoir that is filled with the driving liquid, wherein
the medical-solution reservoir forms a pipe in which the driving liquid discharged by the pump enters from an opening on one end so that the medical solution is pushed to the medical solution administration side from an opening on other end of the pipe, and
a marker is placed between the driving liquid and the medical solution, the marker being used in detecting a pushed quantity of the medical solution pushed by the driving liquid.

2. The medical-solution administration device according to claim 1, wherein the marker is a colored liquid, and
the pipe is transparent.

3. The medical-solution administration device according to claim 2, wherein the marker is provided in the driving liquid.

4. The medical-solution administration device according to claim 3, wherein the marker is a driving liquid made to contain a dye.

5. The medical-solution administration device according to claim 1, wherein the marker is a magnetic marker.

6. The medical-solution administration device according to claim 1, wherein between the driving liquid and the medical solution is filled an isolating fluid including oil that prevents mixing of the driving liquid and the medical solution.

7. The medical-solution administration device according to claim 1, wherein the pipe is folded.

8. The medical-solution administration device according to claim 7, wherein the pipe is wound in a coil shape.

9. The medical-solution administration device according to claim 8, wherein the pipe has a uniform wound diameter.

10. The medical-solution administration device according to claim 8, wherein the driving liquid reservoir is disposed inside an internal space formed by the pipe wound in a coil shape.

11. The medical-solution administration device according to claim 1, wherein the pump is a variable flow rate pump.

12. The medical-solution administration device according to claim 11, wherein the variable flow rate pump is an electro-osmotic flow pump.

13. The medical-solution administration device according to claim 1, wherein the medical-solution reservoir includes a scale that indicates an amount of movement of the marker.

14. The medical-solution administration device according to claim 1, further comprising
a sensor that detects movement of the marker; and
a control unit that, based on a detection result of the sensor, controls the pump for controlling the pushed quantity of the medical solution.

15. The medical-solution administration device according to claim 14, further comprising a memory unit that stores therein a temporal profile of medical solution administration, wherein
the control unit controls a medical-solution-administration quantity per unit time corresponding to the temporal profile.

16. The medical-solution administration device according to claim 14, wherein the sensor is a line image sensor.

17. The medical-solution administration device according to claim 14, wherein the sensor is a magnetic line sensor.

18. The medical-solution administration device according to claim 1, further comprising
a biological information obtaining unit that obtains biological information of a target for administering the medical solution; and
a control unit that, based on an obtaining result of the biological information obtaining unit, controls a pushing quantity from the pump.

19. The medical-solution administration device according to claim 18, further comprising a memory unit in which a relation between the biological information and the medical-solution-administration quantity per unit time is stored, wherein
based on the relation stored in the memory unit, the control unit obtains a medical-solution-administration quantity per unit time corresponding to biological information obtained by the biological information obtaining unit and controls the pushing quantity from the pump in such a way that the pushing quantity is equal to the medical-solution-administration quantity per unit time.

20. The medical-solution administration device according to claim 19, further comprising
a sensor that detects movement of the marker; and
a control unit that, based on a detection result of the sensor, performs feedback control to maintain the medical-solution-administration quantity per unit time.

21. The medical-solution administration device according to claim 20, further comprising a memory unit that stores therein a temporal profile of medical solution administration, wherein
based on the biological information obtained by the biological information obtaining unit, the control unit controls a medical-solution-administration quantity per unit time corresponding to the temporal profile.

22. The medical-solution administration device according to claim 21, wherein the control unit controls a medical-solution-administration start time according to the temporal profile based on the biological information obtained by the biological information obtaining unit.

23. The medical-solution administration device according to claim 1, further comprising a control unit that controls a pushing quantity from the pump, wherein
the control unit has an initial setting mode used in discharging the medical solution at a high velocity while delivering the medical solution for first time.

24. The medical-solution administration device according to claim 1, wherein the driving liquid reservoir and the medical-solution reservoir are detachable and, in an assembled condition, implantable in a human body.

25. The medical-solution administration device according to claim 1, further comprising
a retainer that retains the medical-solution administration device to outside of a biological object; and
a catheter that guides the medical solution discharged from the medical-solution reservoir to an affected area inside a biological object, wherein
the medical-solution administration device is portable.

26. The medical-solution administration device according to claim 1, wherein a maximum quantity of the driving liquid delivered from the driving liquid reservoir is smaller than a medical solution quantity filled in the medical-solution reservoir.

27. The medical-solution administration device according to claim 1, wherein a driving liquid quantity filled in the driving liquid reservoir is smaller than a medical solution quantity filled in the medical-solution reservoir.

28. The medical-solution administration device according to claim 1, wherein, from a driving liquid quantity filled in the driving liquid reservoir, a liquid quantity suppliable to the pump is smaller than a medical solution quantity filled in the medical-solution reservoir.

29. The medical-solution administration device according to claim 1, further comprising
a spacer that is disposed flowably between the medical solution and the driving liquid; and
a blocking member that blocks flowing of the spacer in a flow path that is beyond a delivery side end of the medical-solution reservoir and outside of a biological object.

30. The medical-solution administration device according to claim 1, further comprising
a magnetic bead that is disposed between the medical solution and the driving liquid; and
a holding unit that holds the magnetic bead in a flow path that is beyond a delivery side end of the medical-solution reservoir and outside of a biological object.

31. The medical-solution administration device according to claim 30, wherein the holding unit includes a blocking member that blocks flowing of the magnetic bead.

32. The medical-solution administration device according to claim 1, further comprising
a detecting unit that detects one or more of a driving liquid remaining quantity in the driving liquid reservoir, a flow rate of the driving liquid from the pump, and a flow rate of the medical solution from the medical-solution reservoir; and
a control unit that, based on a detection quantity of the detecting unit, performs control to stop the pump from discharging the driving liquid so that the driving liquid does not flow to a tip side of a flow path that is beyond a delivery side end of the medical-solution reservoir and outside of a biological object.

33. The medical-solution administration device according to claim 1, wherein
the marker is a spacer that is disposed flowably between the medical solution and the driving liquid,
a stop is disposed for blocking flowing of the spacer in the flow path beyond a delivery side end of the medical-solution reservoir and outside of a biological object, and
a driving liquid quantity delivered from the driving liquid reservoir is smaller than a medical solution quantity filled in the medical-solution reservoir.
